(19) Europäisches Patentamt
European Patent Office
Office européen des brevets

(11) Publication number: **0 316 306 B1**

(12) **EUROPEAN PATENT SPECIFICATION**

(45) Date of publication of patent specification: **15.12.93**

(51) Int. Cl.5: **G01N 33/72**, C12P 21/00, C12N 5/00, C12N 15/00

(21) Application number: **89100369.1**

(22) Date of filing: **17.10.85**

(60) Publication number of the earlier application in accordance with Art.76 EPC: **0 185 870**

The file contains technical information submitted after the application was filed and not included in this specification

# (54) Monoclonal antibodies special for HB A1c.

(30) Priority: **29.10.84 US 665811**
**08.08.85 US 763193**
**27.09.85 US 779730**
**27.09.85 US 779731**

(43) Date of publication of application:
**17.05.89 Bulletin 89/20**

(45) Publication of the grant of the patent:
**15.12.93 Bulletin 93/50**

(84) Designated Contracting States:
**AT BE CH DE FR GB IT LI LU NL SE**

(56) References cited:
**EP-A- 0 201 187**
**DE-A- 3 439 610**
**US-A- 4 247 533**
**US-A- 4 478 744**

**CLINICAL CHEMISTRY vol.27,no.9,1st.September 1981,pages 1580-1585,Easton,Pennsylvania,US;G.S.David et al.:"The hybridoma-An immunochemical laser".**

(73) Proprietor: **MILES INC.**
**One Mellon Center**
**500 Grant Str.**
**Pittsburgh, PA 15219-2502(US)**

(72) Inventor: **Knowles, William, Dr.**
**101 Sleeping Giant Drive**
**Hamden, CT 06518(US)**
Inventor: **Marchesi, Vincent, Dr.**
**179 Prospect Avenue**
**Guilford, CT 06437(US)**
Inventor: **Haigh, Wallace**
**189 Ouinnipiac Avenue**
**North Haven, CT 06473(US)**

(74) Representative: **Dänner, Klaus, Dr. et al**
**Bayer AG**
**Konzernverwaltung RP**
**Patente Konzern**
**D-51368 Leverkusen (DE)**

**EP 0 316 306 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

BIOLOGICAL ABSTRACTS
vol.76,1983,G.STAMATOYANNOPOLUS et al.:"Monoclonal antibodies specific for globin chains & blood,vol.61,no.3,1983 ,pages 530-539

## Description

BACKGROUND OF THE INVENTION

This invention relates to the determination of the glucosylated form of hemoglobin known as Hb Alc. The determination of the extent of glucosylation of hemoglobin in an individual's blood provides a useful index of glucose level control in diabetics. Monoclonal antibodies are provided which recognize specifically the glucosylated N-terminal peptide residue in Hb Alc.

Hemoglobin is a protein tetramer made up of four chains (subunits) of amino acids, each of about 143 units and having a total molecular weight of about 64,000. At one end of the molecule (the $NH_2$-terminus of the beta-subunit) there is a valine unit which can react with glucose. The glucosylation of hemoglobin occurs by a non-enzymatic reaction involving glucose and the alpha-amino group of valine. Following a Schiff base formation between the reactants, the glucose undergoes an Amadori rearrangement forming 1-deoxyfructo-valine. This complex is covalent and essentially irreversible. The glucosylation reaction is governed by the concentration of the reactants, e.g., hemoglobin and glucose. In a normal (non-diabetic) individual approximately 3% of the total hemoglobin is glucosylated. Hemoglobin tetramers with a 1-deoxyfructo-valine on the N-terminus of a beta-chain are identified as being glucosylated or $A_{1c}$ hemoglobin.

Glucose levels in diabetics are sufficiently high to increase the rate of glucosylation in direct dependence upon the glucose level in the blood, which reflects the severity of the diabetic condition. With hemoglobin, the $A_{lc}$ level is raised to about 5-12%. Since the circulating life span of hemoglobin is about 120 days, a glucosylated hemoglobin measurement will give a value which reflects an average glucose level for that period. Notably a meal high in glucose will not be reflected in a high glucosylated hemoglobin or serum albumin level. Thus, measurement of the glucosylated hemoglobin content gives a truer picture of the average circulating glucose levels and thus a truer picture of the long term condition of the patient.

U.S. Patent No. 4,247,533 discloses an analytical technique wherein antibodies to Hb $A_{1c}$ were reportedly raised in a special sheep by injection of Hb $A_{1c}$ and absorbed with nonglucosylated hemoglobin to provide polyclonal antibodies which distinguished between Hb $A_{1c}$ and nonglucosylated Hb. Such antibodies then form the basis for a test to determine the proportion of glucosylated hemoglobin in a sample. The test, however, requires an appropriately immunized sheep and antibody absorptions to attain the proper specificity. It is, therefore, costly and difficult to produce specific polyclonal antibodies. The antibody preparations produced by this absorption approach are reported to be of low titer and affinity. The reproducibility of this approach is also open to question since there are no recent reports describing its use for the analysis of clinical samples of human hemoglobin.

Another attempt to obtain antibodies specific for Hb $A_{lc}$ is found in U.S. Patent No. 4,478,744. These workers substituted a synthetic peptide immunogen for the normal hemoglobin molecule as the immunizing agent. This material was injected into an animal which mormally does not have $HbA_{lc}$ in its bloodstream e.g., sheep. The synthetic peptide immunogen comprised a glucosylated peptide residue having an amino acid sequence corresponding to between the first 4 to 10 amino acids in the N-terminal hemoglobin sequence. Subsequent investigations, reported hereinbelow, have found that the sheep polyclonal antiserum raised against the synthetic peptide immunogen has no detectable specificity for the glucosylated form, Hb $A_{1c}$.

General methods for the preparation of monoclonal antibodies are well described in the literature, including David et al., Clinical Chemistry Vol. 27, No. 9 (1981) pages 1580-1585.

## Definitions

| Amino Acid | Abbreviation |
|---|---|
| Arginine | Arg |
| Aspartic Acid | Asp |
| Glutamic Acid | Glu |
| Lysine | Lys |
| Serine | Ser |
| Asparagine | Asn |
| Glutamine | Gln |
| Glycine | Gly |
| Proline | Pro |
| Threonine | Thr |
| Alanine | Ala |
| Histidine | His |
| Cysteine | Cys |
| Methionine | Met |
| Valine | Val |
| Isoleucine | Ile |
| Leucine | Leu |
| Tyrosine | Tyr |
| Phenylalanine | Phe |
| Tryptophan | Trp |

The present invention enaldes the high specific determination of glucosylated proteins such as glucosylated hemoglobin and albumin, and particularly Hb $A_{1c}$ in biological fluids such as blood. Monoclonal antibodies raised against the synthetic glucosylated N-terminal peptide residues appearing in Hb $A_{1c}$ have been found to bind specifically to such residues in the glucosylated beta-subunit of hemoglobin. The antibodies can be prepared in a variety of manners following conventional monoclonal techniques. Principally, the antibodies are prepared against a synthetically derived immunogen comprising the desired glucosylated N-terminal peptide residue chemically linked to an immunogenic carrier, the glucosylated peptide having at least 2, and preferably from about 5 to 15, amino acid units corresponding to Hb $A_{1c}$. The resultant antibodies are specific for the glucosylated synthetic peptide and the corresponding exposed epitope in the hemoglobin $A_{1c}$ molecule.

BRIEF DESCRIPTION OF THE DRAWINGS

The drawings are graphs presenting data from experiments described in the Examples below relating to the immunodetection of Hb $A_{1c}$.

Fig. 1 is a plot depicting the inhibition of Ab-3 binding to $A_{1c}$ by glycopeptide 1 (PEPTIDE 1). Antibody was preincubated with glycopeptide before transfer into an $A_{1c}$ coated microtiter plate. The monoclonal antibody that binds to $A_{1c}$ was detected using a secondary antibody-enzyme. The results are plotted in Fig. 1 as a percent inhibition where 0% inhibition is the value obtained with no competitor. The 0 - 0 line is from an identical peptide that lacks the carbohydrate, indicating the carbohydrate is essential for antibody binding. All points are the mean of triplicate measurements.

Fig. 2 is a plot depicting the inhibition of Ab-3 binding to $A_{1c}$ by glycopeptide 3 (PEPTIDE 3). The competitive experiment was done as described for Fig. 1.

Fig. 3 is a plot depicting the inhibition of Ab-3 and Ab-4 by glycopeptide 4 (PEPTIDE 4) for $A_{1c}$ binding. The competition experiment was conducted as described for Fig. 1.

Fig. 4 is a typical standard curve using optimal assay conditions. The whole blood standard was prepared using different ratios of denatured whole blood from a diabetic having 12.66% $A_{1c}$ as measured by HPLC ion exchange with whole blood from a normal donor (3.83% $A_{1c}$). All points of triplicate measurements are plotted.

Fig. 5 is a standard curve using a synthetic peptide standard. The assay was performed as described for Fig. 4, except that instead of using whole blood, different amounts of synthetic glycopeptide were used as the competitor. All values of triplicate determinations were plotted.

Fig. 6 is a plot depicting a comparison of the immunoassay method with the boronate affinity method for donors. The mean of triplicate determinations are plotted for the immunoassay coordinate.

Fig. 7 is a plot demonstrating the exposure of the Hb Alc epitope under varying denaturation conditions.

Fig. 8 is a plot depicting the results of immunizing a sheep with the synthetic glycopeptide of Example 1(b).

Fig. 9 is a plot demonstrating that mouse monoclonal antibodies are specific for $A_{1c}$ hemoglobin.

## DESCRIPTION OF THE PREFERRED EMBODIMENTS

The immunoassay determination of Hb Alc using the present antibody reagent can follow essentially any conventional technique. Such include the more classical techniques such as immunodiffusion, immunoelectrophoresis, agglutination techniques, and complement fixation, as well as more current techniques involving the use of specifically detectable labels such as radioimmunoassay and nonradioisotopic methods. The performance of an immunoassay for Hb Alc employing the present invention involves the essential steps of treating the aqueous test sample involved to effectively denature a significant amount of any such protein therein to expose the desired linear peptide epitope, contacting the denatured sample with the antibody reagent, and determining binding of the antibody reagent to such protein. The determination step will of course vary according to the basic immunoassay technique involved. A common technique for making this determination involves the use of a labeled reagent which interacts with either the analyte or antibody reagent and is employed in a manner to indicate the formation of immune complex between analyte and the antibody reagent or to compete with such formation.

The latter techniques can be practiced in a wide variety of formats such as the competitive binding format in which a labeled reagent is made to compete with the protein analyte for binding to the antibody reagent. The amount of labeled reagent bound to the antibody reagent, or the free-species, consisting of the labeled reagent which is not so bound, is measured appropriately and can be functionally related to the amount of protein analyte in the sample. Since the antibody reagent of the present invention is directed to a linear epitope in the protein analyte, the labeled reagent can be a labeled form of the denatured protein or a denatured fragment thereof, or, as would be preferred, a labeled form of a peptide residue comprising the linear epitope sequence of amino acids. The latter, preferred reagent can be prepared by available synthetic peptide methods and apparatus and does not require isolation, purification, and denaturation of the protein molecule itself.

Another useful immunoassay technique for the detection of protein analytes is that known as the sandwich technique. In this method, one would employ two sets of antibody reagents, one of which would be labeled and the other would be adapted to effect separation of ultimately labeled first antibody reagent bound to the protein analyte from that which is unbound. The unlabeled second antibody reagent typically is in an immobilized or immobilizable form as is known in the art.

In radioimmunoassays, the free-species and bound-species must be physically distinguished or separated in order to measure the label since the signal generated by the label is qualitatively the same in both species. Such a technique is known in the art as heterogeneous because of the phase separation requirement. Other heterogeneous immunoassay techniques are known including enzyme-labeled immunoassays, sometimes referred to as ELISA techniques (see U.S. Pat. No. 3,654,090), and fluorescent

immunoassays (see U.S. Pat. Nos. 4,201,763; 4,133,639 and 3,992,631).

Fairly recently, numerous immunoassay techniques have been developed which obviate the separation step through the use of a label whose detectable signal is modulated upon binding of the labeled reagent by a binding partner, e.g., antibody. Such techniques have become known as homogeneous and when available are preferred for use in the present invention because separations are not required and radioisotopes are not involved. Some such techniques are fluorescence quenching and enhancement (see U.S. Pat. No. 4,160,016), energy transfer immunoassay (see U.S. Pat. No. 3,996,345), and double antibody steric hindrance immunoassay (see U.S. Pat. Nos. 3,935,074 and 3,998,943). Particularly preferred homogeneous immunoassay techniques are those employing a label which is a participant in an enzyme-catalyzed reaction. Examples are the substrate-labeled immunoassay (see U.S. Pat. No. 4,279,992 and U.K. Patent Spec. 1,552,607), the prosthetic group (FAD)-labeled immunoassay (see U.S. Pat. No. 4,238,565), the enzyme modulator-labeled immunoassay, e.g., using inhibitor labels (see U.S. Pat. Nos. 4,134,972 and 4,273,866), and enzyme-labeled immunoassay (see U.S. Pat. No. 3,817,837).

The monoclonal antibody reagent of the present invention is characterized by its specific binding affinity for the linear peptide epitope in Hb Alc. Therefore, as used herein the term "antibody reagent" will refer to any material however obtained which comprises a monoclonal antibody combining site specific for such peptide epitope. Such expression therefore includes whole antibodies as well as appropriate fragments or polyfunctionalized forms thereof. When in the form of whole antibody, it can belong to any of the classes and subclasses of known immunoglobulins, e.g., IgG, IgM, and so forth. Any fragment of any such immunoglobulin which retains specific binding affinity for the peptide epitope can also be employed, for instance, the fragments of IgG conventionally known as Fab, Fab′, and $F(ab')_2$. In addition, aggregates, polymers, derivatives, conjugates, and hybrids of immunoglobulins or their fragments can be used where appropriate.

The immunoglobulin source for the monoclonal antibody reagent can be obtained in any available monoclonal techniques. Thus, the immunoglobulins can be obtained through somatic cell hybridization, such resulting in what are commonly referred to as monoclonal antibodies.

Hybridoma cell lines are raised to produce antibodies only against the linear peptide epitope portion of the protein molecule rather than to the entire protein and such cell lines and their antibodies are screened to identify and isolate those monoclonal antibodies which will react selectively with the desired epitope.

In one method to produce such antibodies, a fragment of the protein chain, corresponding to and comprising the naturally occurring linear peptide epitope sequence, is coupled to a protein carrier and injected into a laboratory animal to elicit an immune response. Alternatively, the immunogen can comprise a linearized or denatured form of the protein or a fragment thereof. Lymphocytes such as spleen cells from the immunized animal are fused with myeloma cells to produce hybridomas which are cultured and screened for production of monoclonal antibodies. The monoclonal antibodies are screened for those selective to the peptide epitope and the particular cell line is cloned for use in producing further quantities of the monoclonal antibody.

To produce antibodies against a synthetic peptide immunogen in the laboratory animal, e.g., BALB/c mice, rats or the like, a peptide comprising the desired epitope will be produced and isolated from the naturally occurring protein or will be chemically synthesized and purified. Such a protein fragment will include all of the critical amino acid units of the desired epitope and can include additional amino acid units, some or all of which optionally will correspond to the sequence of amino acids in the protein of interest.

To ensure that the epitope-containing peptide fragment is optimally antigenic, it can be advantageously coupled in multiples to an immunogenic carrier material. The immunogenic carrier material can be selected from any of those conventionally known having functional groups available for coupling to the peptide residue. In most cases, the carrier will be a protein or polypeptide, although other materials such as carbohydrates, polysaccharides, lipopolysaccharides, nucleic acids, and the like of sufficient size and immunogenicity can likewise be used. For the most part, immunogenic proteins and polypeptides will have molecular weights between 4,000 and 10,000,000, preferably greater than 15,000, and more usually greater than 50,000. Generally, proteins taken from one animal species will be immunogenic when introduced into the blood stream of another species. Particularly useful proteins are albumins, globulins, hemocyanins, glutelins, and the like. Further reference for the state-of-the-art concerning conventional immunogenic carrier materials and techniques for coupling haptens thereto may be had to the following: Parker, Radioimmunoassay of Biologically Active Compounds, Prentice-Hall (Englewood Cliffs, New Jersey USA, 1976); Butler, J. Immunol. Meth. 7:1.-24(1974); Weinryb and Shroff, Drug Metab. Rev. 10:271-283(1974); Broughton and Strong, Clin. Chem. 22:726-732(1976); and Playfair et al, Br. Med. Bull. 30:24-31(1974).

The number of epitopes coupled to a given immunogenic carrier material will be limited only by the number of available coupling sites on the carrier and can be as high as several thousand in the case of

certain high molecular weight synthetic polypeptides such as polylysine. The epitopic density on a particular carrier will depend upon the molecular weight of the carrier and the density of available coupling sites. Optimal epitopic densities, considering the ease and reproducibility of synthesis of the immunogen and antibody response, fall between about 10% and about 50% of the available coupling groups on the carrier involved.

The peptide fragments will be coupled to the carrier material by any convenient coupling method. Functional groups on the native amino acids in the fragment or functional groups introduced by chemical modifications of the fragment will normally be used to couple directly or through bifunctional coupling agents to functional groups on the carrier. It will be preferred to design the peptide fragment to have a single uniquely reactive functional group for obtaining unambiguous coupling to the carrier.

Monoclonal antibodies raised against the synthetic glucosylated N-terminal peptide residues appearing in Hb Alc have been found to bind specifically to such residues in the glucosylated beta-subunit of hemoglobin. The antibodies can be prepared in a variety of manners following conventional monoclonal techniques. Principally, the antibodies are prepared against a synthetically derived immunogen comprising the desired glucosylated N-terminal peptide residue chemically linked to an immunogenic carrier, the glucosylated peptide having at least 2, and preferably from about 5 to 15, amino acid units corresponding to Hb Alc. The resultant monoclonal antibodies are specific for the glucosylated synthetic peptide and the corresponding exposed epitope for the hemoglobin Alc molecule.

Monoclonal antibodies specific to Hb Alc found in human blood are secreted by hybridomas derived from fusion of myeloma cells and lymphocytes taken from an animal that had been immunized with a synthetic peptide immunogen. The synthetic peptide immunogen will preferably be of the formula:

$$[\text{Glyco-(NH)Val-His-AA-R}]_n\text{—Carrier}$$

wherein Glyco-(NH)Val represents a nonenzymatically glucosylated valine residue, His represents the second amino acid in the native beta-subunit Hb sequence, AA is a bond or one or more amino acid residues, R is an appropriate linking group, Carrier is an immunigenic carrier material, and n (the epitopic density) is on the average from 1 to the number of available coupling sites on the Carrier. Linking group R can consist of any desired coupling reagent and AA can comprise one or more additional amino acid residues corresponding to the carbohydrate-bearing N-terminus of the beta-subunit of human hemoglobin. For example, -AA- can be selected from the following amino acid sequence or any continuous fragment thereof which begins with the leucine unit:
-Leu-Thr-Pro-Glu-Glu-Lys-. In addition, linking group R can consist of additional amino acid units not found in normal human hemoglobin but which can be conveniently added by peptide synthesis methods and can serve as useful functional groups for coupling to the carrier materials. A particularly useful linking group is -Tyr-Tyr-Cys which provides a unique thiol group for controllably coupling the glucosylated peptide unit to carrier materials.

The monoclonal Hb Alc antibody of the present invention is principally characterized by its specificity for binding the glucosylated form of the N-terminal peptide sequence of the beta-subunit of human hemoglobin. This glucosylated residue is the distinguishing structural feature of Hb Alc. An antibody of the present invention requires an epitope or determinant site comprising minimally the 1-deoxyfructosyl carbohydrate unit, formed upon Amadori rearrangement of the reaction product between glucose and the terminal amine, and a peptide sequence extending therefrom comprising at least one of the amino acid units of the Hb Alc N-terminal sequence in the position corresponding to the native Hb Alc sequence. The other amino acid units in the peptide sequence characterizing the epitope may be the same or different as those appearing in the native Hb Alc sequence. In this way, the epitope is characterized by at least two contact or binding sites with the antibody which sites are unique to the glucosylated N-terminal Hb Alc sequence. Preferably the antibody will specific bind a glucosylated peptide residue of the formula:
Glyco-(NH)Val-His-AA-
wherein Glyco-(NH)Val and AA are as defined above. Particularly preferred monoclonal antibodies have been found to be specific for the glucosylated dipeptide residue irrespective of the nature of AA. Antibodies with specificity requiring glucosylated peptide sequences of greater length are also obtainable with AA being a sequence of from 1 to 12, preferably 1 to 6, amino acids corresponding to the N-terminus of the beta-subunit of human hemoglobin. Such specificity of the monoclonal antibody enables the specific detection of the exposed glucosylated N-terminal peptide residue in Hb Alc to the substantial exclusion of other glucosylated peptide epitopes on hemoglobin and other proteins and peptides native to the human

bloodstream.

The glucosylated N-terminal peptide residue on the native Hb Alc molecule is made accessible to the monoclonal antibody or a fragment thereof of the present invention by appropriate denaturation or digestion of the protein in the sample to be assayed. An underlying hypothesis for the success of the present invention in obtaining specific antibodies where prior art attempts have failed will now be discussed, but its correctness should not be interpreted as being critical to the inventiveness of the present method.

The N-terminal sequence of the beta-subunit of human hemoglobin is quite similar to the corresponding sequence of mouse hemoglobin, the first four amino acids being identical. Secondly, mouse hemoglobin is glucosylated to approximately the same extent as human hemoglobin. Thus, in the native human hemoglobin molecule the N-terminal sequence of the beta-subunit would not be seen by the mouse as foreign and an immune response would not be expected. This is the logic of the prior art workers who accordingly chose an animal (sheep) that has a quite different hemoglobin protein sequence, and is not glucosylated in the hopes of obtaining an immune response. However, the present invention has revealed that when the glucosylated N-terminal residue is exposed to the mouse immune system in the form of a synthetic peptide immunogen, the epitope is presented in a configuration to which the mouse can respond immunologically. Through somatic cell cloning techniques, hybridomas secreting highly specific antibodies can be isolated. The secreted antibodies will bind to the glucosylated N-terminal peptide reside in the native hemoglobin molecule if it has been exposed sufficiently for interaction with the combining site on the antibody. The manner of exposure of the epitope is discussed in more detail below.

Steric access of the antibody reagent to the epitope can be obtained in any effective manner. Exposure of the epitope in the intact protein is understood to be accomplished by a physical or chemical denaturation or digestion at least in the region of the epitope. Such denaturation or digestion can be localized to the region of the epitope or can involve a more general, or even substantially complete denaturation of the tertiary, and additionally the secondary, structure of the protein, or partial or complete digestion of the protein.

Denaturation can be accomplished in a variety of ways including conventional treatment of the protein by physical means such as heat, sonication, high or low pH and, as is preferable, chemical denaturation by digestion or interaction with a chaotropic agent or chaotrope in solution. Useful chaotropic agents include, without limitation, guanidine, urea, and various detergents such as sodium dodecylsulfate (SDS) and others, without limitation, including deoxycholate and certain bile salts, 3-(3-cholamidopropyl)-dimethyl-ammonio-1-propanesulfonate, organic solvents such as methanol, propanol, acetonitrile and certain salts such as potassium thiocyanate. Non-ionic detergents such as Triton X®, Tween®, nonidet NP-40® and octylglucosides can also function as protein denaturants. Inclusion of reagents (e.g., mercaptoethanol or dithiotrietol) that reduce disulfide bonds can be effective promoters of the denaturation process. Protein denaturation can be most effectively accomplished if combinations of chemical and/or chemical and physical means are used (e.g., guanidine and heat, guanidine and SDS, or guanidine and dithiothreitol). Particularly strong chaotropes such as guanidine are most preferred. Of course, denaturing conditions which result in substantial aggregation, insolubilization, or precipitation of the protein such that an insignificant amount of the exposed epitope is accessible to the solution for antibody binding will be avoided. A sufficient amount of the denatured protein must remain in solution or suspension in order to obtain useful immunobinding. The extent of solubilization necessary will depend upon the circumstances of the intended or desired binding.

A significant amount of a desired protein in a particular test sample can be denatured to expose the peptide epitope for antibody binding by combining the sample with an aqueous solution of the chaotrope present at sufficient concentration to denature a significant amount of the protein in the resulting aqueous mixture. Where whole blood is the sample, the chaotrope also serves to lyse red blood cells, to release Hb and to inactivate protease. In the case of guanidine, the concentration in the mixture will preferably be greater than about one molar, with about 3 molar concentration being particularly useful. The denaturation process is significantly accelerated by heating the mixture for a short period of time. It has been found that at temperatures below 37°C, denaturation by the chaotrope can take from one to several hours, whereas at temperatures above 50°C sufficient denaturation can be attained in a minute or less. In order to prevent significant denaturation of the antibody and other proteinaceous reagents to be subsequently added to the mixture, the sample-chaotrope mixture will normally be diluted as a separate step or by addition of reagent solutions to a level that the chaotrope is substantially ineffective to denature such reagents, yet will preserve the exposure of the epitope by preventing significant renaturation of the protein of interest. For guanidine, this preferably requires dilution to a concentration less than about 1.0 molar, with about 0.3 molar being particularly preferred.

Non-limiting examples of proteolytic enzymes for use in the present invention for digestion including trypsin, chymotrypsin, proline-specific endoprotease, pepsin and papain. In performing an immunoassay, inhibitors for the proteolytic enzymes, as are known, are added to the assay mixture sufficient to prevent digestion of proteinaceous assay agents.

Specifically, hybridoma cell lines are raised to produce antibodies only against the glucosylated portion of the hemoglobin molecule rather than to the entire protein and such cell lines and their antibodies are screened to identify and isolate those monoclonal antibodies which will thereafter react selectively with the glucosylated Hb Alc epitope.

To produce such antibodies, a fragment of the protein chain, corresponding to the naturally occurring glucosylated peptide sequence, is coupled to a protein carrier and injected into a laboratory animal to elicit an immune response. Lymphocytes such as spleen cells from the immunized animal are fused with myeloma cells to produce hybridomas which are cultured and screened for production of monoclonal antibodies. The monoclonal antibodies are screened for those selective to the glucosylated peptide epitope and the particular cell line is cloned for use in producing further quantities of the monoclonal antibody.

To produce antibodies in the laboratory animal, e.g., BALB/c mice, rats or the like, a glucosylated hemoglobin fragment must be either produced and isolated from naturally occurring human hemoglobin or be chemically synthesized and purified. The hemoglobin fragment should include the 1-deoxyfructose residue and at least 2 amino acid units, preferably 3, 4, 5 or even more, corresponding to the N-terminus of the beta-subunit of hemoglobin (valine-histidine). Advantageously it includes about 5 to 15 and preferably about 7 to 10 units.

To ensure that the glucosylated peptide fragment is optimally antigenic it can be advantageously coupled to a carrier material comprising a large immunogenic molecule such as bovine serum albumin (BSA) or keyhole limpet hemocyanin (KLH). The fragment should also carry the natural rearranged adduct of the glucose-valine reaction which can be present from the outset, as in the case of the isolated naturally occcurring hemoglobin fragment, or, preferably, can be formed on the synthetic peptide during ts synthesis or before coupling the peptide to the large protein carrier. The carrier can be added in any manner which does not destroy the antigenicity of the fragment.

The glucosylated fragment can be produced by chemical or enzymatic digestion of naturally occurring Hb, e.g., Alc. This fragment can be coupled to a carrier using classical coupling procedures, e.g., glutaraldehyde or carbodiimide, and the conjugate used as an immunogen.

A preferred manner of chemically synthesizing a portion of the known hemoglobin sequence involves the addition of one or more amino acid units (not found in the normal sequence) for optimizing its antigenicity and coupling properties. In this case, the final unit carries a thiol (SH) group by which it can be coupled to the ligand in a conventional manner, as by reaction with a bifunctional linking reagent such as m-maleimidobenzoyl N-sulfosuccinimide ester (MBS).

In accordance with a preferred embodiment, to the lysine end of a synthetic Hb fragment carrying the eight units
$NH_2$-valine-histidine-leucine-threonine-proline-glutamic acid-glutamic acid-lysine-COOH there were added tyrosine, tyrosine and cysteine, resulting in an 11-unit cysteine-terminated peptide.

This can be glucosylated in conventional manner by nonenzymatic reaction with glucose. The glucopeptide thereafter is coupled to a large carrier to produce the antigen which is administered to produce the antibodies. Lymphocytes from the animal which produce antibody to the glucosated peptide epitope are then fused in conventional manner to produce hybridomas which are cloned and those producing monoclonal antibodies of the desired specification are further subcloned. The cell line(s) whose monoclonal antibodies show the greatest selectivity for the glucosylated epitope, as opposed to non-glucosylated Hb, are then propagated and the antibodies harvested. Reviews of such monoclonal antibody techniques are found in Lymphocyte Hybridomas, ed. Melchers et al, Springer-Verlag (New York 1978), Nature 266:495 (1977), Science 208:692 (1980), and Methods in Enzymology 74(Part B): 3-46 (1981).

The antibodies can then be used in conventional manner to react with blood samples containing unknown quantities of glucosylated Hb and the extent of reaction can be compared with calibrated standards to determine the extent of glucosylation. The read-out can be by fluorescence, by immunoassay, or the like, by joining suitably readable groups to the monoclonal antibodies in known manner without loss of their binding power for the glucosylated epitope in Hb Alc.

Alternatively, an assay based on a reagent test strip can be run in which a carboxyl-carrying material such as carboxylmethyl-cellulose is coated onto a strip of wood or plastic. Then the strip is dipped into the lysed and denatured unknown blood sample, thereby adsorbing the hemoglobin, glucosylated or not. The strip is then dipped into a solution of the monoclonal antibodies, suitably labeled (e.g., enzyme, fluorescence, cofactors, etc.) at a site which does not interfere with binding to the Hb Alc epitope. The amount of

antibody bound is determined by the amount of label on the strip and is an indication of the amount of glucosylated Hb in the unknown sample. The attachment of the label and its read-out are effected in conventional manner.

Linking group R in the formula above can be essentially any convenient and stable structure. Such linking group R will usually be in the form of an aliphatic chain comprising between 1 and about 20 atoms, excluding hydrogen, and including heteroatoms such as nitrogen, oxygen, and sulfur. The glucosylated residue can be joined through a variety of groups to form linking chain R, including methylene, ether, thioether, imino, and the like. One skilled in the art will have a wide variety of linking groups from which to choose to prepare the immunogen. Normally, the glucosylated peptide will be prepared terminating in a functional group such as amino, carboxyl, thiol, hydroxyl, or maleimido which is active in a coupling reaction to an appropriate group in the carrier molecule.

The present invention will now be illustrated, but is not intended to be limited, by the following specific examples.

EXAMPLE 1: Preparation and characterization of Antibodies to the Glycopeptide Epitope in Hb A1c

a) An 11-amino acid peptide comprising the 8 N-terminal units of beta-hemoglobin plus two units of tyrosine plus a unit of cysteine was synthesized according to Gutte, B. and R.B. Merrifield; J. Am. Chem. Soc., 91:2,501(1969), giving the following peptide:

$NH_2$-valine-histidine-leucine-threonine-proline -glutamic acid-glutamic acid-lysine-tyrosine-tyrosine-cysteine-COOH.

To glucosylate this peptide, 200 mg of this purified peptide is reacted with 0.25 molar glucose in 20 ml of anhydrous pyridine for 48 hours at room temperature in the dark. The mixture is dried in vacuum. The resulting syrup is resuspended in 20 millimolar potassium phosphate, pH 2.95 and purified by HPLC.

The glucopeptide-bearing fractions are dissolved in 0.1M triethylammonium acetate pH 8.5 and chromatographed over Affigel-601 boronate affinity resin (Biorad), whereby the glucopeptide is selectively adsorbed. The resin is washed with 0.1M triethylammonium acetate pH 8.5 and the glucopeptide eluted with 0.lM triethylammonium acetate pH 5.0. The eluate is lyophilized.

The product is resuspended in 1 ml of water, reacted with a 500 fold molar excess dithiothreitol (to restore the SH group of the cysteine) and the reduced peptide repurified by HPLC, and lyophilized. This glucopeptide is stored at -20 ° C under $N_2$ until further use.

b) a KLH-MBS conjugate, as previously described, Lerner, R. et al, Proc. Natl. Acad. Sci. 78:3403(1981) is reacted with the product of (a) in a 2-fold molar ratio of glucopeptide to maleimide on the carrier, in 50 millimolar (mM) potassium phosphate, pH 7.2, for 1 hour at room temperature.

c) The solution in (b) is mixed with equal volumes of Freund's complete adjuvant to form a water-in-oil emulsion and 200 µg of conjugate is injected into BALB/cBy mice. The mice are boosted at 30 and 60 days, sacrificed, and their spleens used for fusion according to Kohler and Milstein, Nature 256:495-(1975), producing numerous hybridomas. The hybridomas are screened to identify those which produced monoclonal antibodies specific for the glucosylated peptide epitope.

The screening for $A_{1c}$ specific monoclonal antibodies is conducted using an ELISA format, where the antigen is absorbed onto polystyrene microtiter plates (Linbro). The antigens are purified human $A_{1c}$ and non-glucosylated Ao hemoglobin. The $A_{1c}$ is purified from a red blood cell hemolysate using two different chromatographic procedures. The first purification consists of binding glycosylated hemoglobin onto a boronate affinity resin as described by Pierce Chemical Co., Rockford, Illinois, U.S.A. product no. 42,000. Typically 1 to 5 grams of hemoglobin are applied to 100 ml boronate resin, and the bound (glycohemoglobin) fraction elutes as described by Pierce Chemical Co., GlycoTest bulletin, product no. 42,000. The eluted glycohemoglobin fraction is equilibrated in low ionic strength buffer and chromatographed on an ion-exchange resin as described by McDonald, M. et al, J. Biol. Chem., 253:2327-2332-(1978). The $A_{1c}$ "peak" is analyzed by isoelectric focusing and by carbohydrate analysis using the thiobarbituric acid assay and the results confirm that this purification produced ultrapure $A_{1c}$ hemoglobin in that the purified material has both carbohydrate and differed from normal Ao hemoglobin in isoelectric point. Similarly, Ao hemoglobin as purified by its property of not binding to the boronate affinity resin and chromatographing by ion-exchange as the Ao "peak" on the ion-exchange chromatographic purification. The pure $A_{1c}$ and Ao hemoglobins are adsorbed onto separate microtiter plates (2 µg per 100 microliters PBS per well) overnight at 4 ° C. The plates are blocked in 1% BSA in PBS for 60 minutes at room temperature then washed 4 times in PBS. Supernatant from each hybridoma cell line is added to the $A_{1c}$ and Ao plate and incubated at room temperature for 60 minutes. The plates are washed 4 times in PBS and a secondary antibody (rabbit-anti-mouse IgG-peroxidase, Miles Laboratories, Inc., Elkhart, Indiana

U.S.A. at a 1:5000 dilution in 1% BSA in PBS) is applied and is subject to incubation for 60 minutes at room temperature. The plate is washed 4 times in PBS and 200 microliters of a substrate solution added (24.3 mM citric acid, 51.4 M sodium phosphate, pH 5.3 containing 2.2 mM M o-phenylenediamine and 5.2 mM hydrogen peroxide). The reaction is terminated after 20 minutes by adding 50 microliters of 8 M $H_2SO_4$ and the product of the peroxidase reaction is read at 492 mM.

From 200 starting hybridomas producting antibodies against hemoglobin, nine (9) are identified as being specific for the $A_{1c}$ epitope, whereas 191 reacts both with $A_{1c}$ and non-glucosylated hemoglobin. Since pre-immunized mouse serum has no detectable antibody response to Ao or $A_{1c}$ human hemoglobin by the ELISA procedure, the major immune response is against the eight peptide sequence that is shared in common with $A_{1c}$ and Ao. Since the synthetic peptide immunogen consists of eight amino acid residues, of the hemoglobin sequence, the major mouse immune response is directed against the peptide, and not the carbohydrate (191 of the 200 hybridomas reacting both with Ao and $A_{1c}$). As expected, the immunized mouse serum also has broadly cross reacting antibodies reactive both with Ao and $A_{1c}$ suggesting that no specificity for $A_{1c}$ is obtained unless hybridomas are screened for reactivity against $A_{1c}$ and not against Ao hemoglobin. The preferred hybridomas producing antibodies against $A_{1c}$ hemoglobin and not against Ao hemoglobin, were deposited with ATCC identified as ATCC HB 8639 and ATCC HB 8869, deposited on October 11, 1984 and July 10, 1985, respectively.

d) Identification of the peptides that compete with $A_{1c}$ for binding to antibody:

The following peptides are generated by enzyme digestion of the glucosylated 11-amino acid parent peptide

Glyco-Val-His-Leu-Thr-Pro-Glu-Glu-Lys-Tyr-Tyr-Cys (GLYCOPEPTIDE 1):

All peptide fragments are purified by HPLC and quantitated by amino acid sequence. Tryptic digestion of the parent peptide produced

Glyco-Val-His-Leu-Thr-Pro-Glu-Glu-Lys. (GLYCOPEPTIDE 2)

A proline specific endoprotease produces

Glyco-Val-His-Leu-Thr-Pro. (GLYCOPEPTIDE 3)

The peptide Glyco-Val-His-FAD (GLYCOPEPTIDE 4)

wherein the dipeptide is coupled to $N^6$-aminohexyl FAD and then glucosylated is made by the method of Carrico and Johnson, U.S. Pat. No. 4,255,566 and provided by Dr. Kin Yip and Dr. R. Buckler, Ames Division, Miles Laboratories, Inc., Elkhart, Indiana U.S.A.

In a typical competition assay, each peptide 8 nanomoles to 8 picomoles in 100 µl PBS-7.2 mM $Na_2HPO_4$, 2.8 mM $NaH_2PO_4$, 127 mM NaCl, pH 7.4 is incubated with 100 µl monoclonal cell culture supernatant for 60 minutes at room temperature. This mixture is added to a polystyrene microtiter plate coated with 1 µg $A_{1c}$ hemoglobin/well. If the peptide competes with the antibody, the antibody is not free to bind to the immobilized $A_{1c}$. The plate is washed four times with PBS. A second antibody (rabbit anti mouse IgG coupled with horseradish peroxidase) is added for 30 minutes and the plate is washed in PBS. The substrate

(o-phenylenediamine 2.2 mM), and hydrogen peroxide (0.012%) are added and the color produced measured at 492 nm. The quantitation of the product reflects the extent of competition, e.g., no product indicates that the competing peptide totally blocked the antibody from binding to the immobilized $A_{1c}$. The results indicate that all four of the previously described glycopeptides including Glyco-Val-His-FAD are effective competitors. One of the antibodies, Ab-4, is totally blocked from binding to $A_{1c}$ by GLYCOPEPTIDES 1 to 4 (see Fig. 1-3). Another antibody, Ab-3, is blocked by GLYCOPEPTIDES 1 to 3, but not by GLYCOPEPTIDES 4 (see Fig. 1-3).

Peptides lacking the carbohydrate show no competition inhibition suggesting that the carbohydrate is an essential component of the epitope and provides the specificity for the antibodies' recognition of $A_{1c}$ hemoglobin (see Fig. 1).

EXAMPLE 2: Competitive Immunoassay for Hb Alc

This competitive immunoassay is based on the use of a fixed amount of hapten-label (as described in Example 5) that competes with $A_{lc}$ in lysed whole blood for binding to the immobilized antibody. Since the antibody recognizes both the $A_{1c}$ and hapten, the level of $A_{1c}$ in the specimen determines the amount of hapten-label that binds to the antibody. Since the antibody is immobilized, all non-bound reactants can be removed by a simple washing step. The bound label can then be measured and compared to a standard for quantitation of $A_{1c}$ in the original blood samples.

The assay is developed using whole blood as the specimen and can be divided into the steps listed below:

(1) Lysis of cells-denaturation of hemoglobin:

Since the final assay requires less than 0.3 microliters of whole blood, an accurately pipettable volume of blood (5-50 µl from a finger stick or from whole blood) is diluted into a denaturing solution (3M guanidine HCl, 10mM Tris-HCl pH 7.5) and heated to 56 °C for 2 to 15 minutes. Lower temperatures also work, but additional time is required for the complete denaturation of the sample. The denaturation (a) inactivates the clotting mechanisms if samples are not prepared in anticoagulants; (b) lyses the red cells; (c) denatures proteases, enzymes etc., and optimally exposes the $A_{1c}$ epitope on hemoglobin; (d) appears to either sterilize or inhibit the growth of microorganisms in the denatured blood sample even if the sample is non-aseptically prepared and handled (e.g., blood from a finger stick) and (e) results in a stable clinical sample that can be stored for days at room temperature without effect on the final assay.

(2) Dilution and Competition.

An aliquot of the denatured whole blood is pipetted into a 10 fold volume of buffer containing the hapten-label. This effectively dilutes the hemoglobin to the proper concentration for the assay and dilutes the denaturant to a low concentration so as not to perturb the antibody or enzyme activity. The antibody coated bead is then added for a specified amount of time during which the antibody binds either the $A_{1c}$ hemoglobin or the hapten-HRP.

(3) Wash and Read.

Following the competitive incubation, the bead is washed and the label read following the addition of an appropriate substrate. The signal is then compared to a standard and the amount of $A_{1c}$ present in the original whole blood sample determined.

The details of the assays used are summarized below:

Bead Coating Procedure:

Polystyrene beads (1/4 inch diameter with specular finish) are obtained from Precision Ball Company, Chicago, Illinois, U.S.A. Lots are screened for beads that provided the lowest variability in multiple immunoassay determinations of the same sample. Prior to coating, beads are washed with absolute methanol followed by water. The methanol wash seemed to significantly lower the correlation of variation for multiple determinations of the same sample. An antibody solution (5 µg antibody/100 µl in 0.2M sodium borate, pH 8.5, 0.02% sodium azide) is then added to the damp beads and the beads rotated overnight at 4 °C. The beads are then washed, blocked with 1% BSA in PBS containing 0.02% sodium azide. Typically, 500 to 1000 beads are coated at one time and used for a period of weeks with no evidence of loss of antibody activity. Coating experiments with radioactive antibody indicate that 0.5 µg of antibody binds per bead.

The beads are used in this immunoassay only because of their property of binding relatively high amounts of protein. The hydrophobic absorption of protein onto polystyrene is convenient, but certainly could be replaced by one of several procedures where proteins are covalently attached to polystyrene, functionalizd resins, or silica. The polystyrene can also be in the form of a tube or cuvette.

The working protocol is summarized as follows:

(a) Dilute 50 microliters whole blood into 1.0 ml denaturing solution (3M guanidine-HCl, 10mM Tris pH 7.5), heat to 56 °C, 15 minutes, dilute again 100 µl into 1.0 ml denaturant.

(b) Add 50 microliters of the above solution to 0.5 ml phosphate buffered saline (PBS) pH 7.5 containing hapten-HRP. The incubations, washings and enzymatic reactions are conveniently conducted in 48-well polystyrene tissue culture plates.

(c) Add antibody coated bead and incubate 30 minutes at ambient temperature with rocking.

(d) Wash beads with buffer (PBS) (usually 3-l ml changes).

(e) Add o-phenylenediamine substrate and hydrogen peroxide.

(f) Stop the reaction and read the product after 20 minutes. The above assay is used in establishing the clinical data described below. The standard curve is shown is Fig. 4. Competition using GLYCOPEPTIDE 1 is shown in Fig. 5. Evaluation of normal and diabetic donors is shown in Fig. 6. The boronate affinity determination are performed exactly as described by Pierce Chemical Co. (GlycoTest, product no. 42,000).

EXAMPLE 3: Optimal exposure of the A1c epitope.

Optimal reactivity of the human $A_{1c}$ epitope is seen following treatment of the native hemoglobin (in whole blood or hemolysate) with procedures or reagents which expose the epitope to the antibody combining site. The optimal exposure of the epitope can be accomplished by a physical denaturation (heat, sonication, etc.), by a chemical procedure involving classical denaturants (urea, guanidine, SDS, protease) or by a combination of physical and chemical procedures. Most effective is a procedure in which whole blood (50 microliters) is added to a 1 ml solution of 3M guanidine hydrochloride, 10 mM Tris-HCl, pH 7.4 and heated to 56°C for greater than one minute. The resulting sample works optimally in subsequent immunoassays for the $A_{1c}$ epitope. The solution can be diluted ten fold in buffer, effectively diluting the guanidine to 0.3M, a concentration that has little if any effect on normal antibody-antigen interactions and enzyme activities, providing a suitable media for subsequent immunoassays.

The competitive immunoassay of example 2 is used. The competitor is the Alc present in whole blood from a diabetic. The whole blood sample is placed in 3.0 M guanidine at 20°C, 37°C or 56°C for periods of time from 0-320 minutes. The results (Fig. 7) show that with time at 20°C or 37°C the pitope is exposed and thus effectively competes with the hapten-HRP conjugate. At 56°C the epitope is maximally exposed after 5 minutes, the earliest point determined in this assay. The results show that in the native hemoglobin Alc tetramer the epitope is buried and inaccessible and does not compete with the linear synthetic glycopeptide-HRP conjugate. However if hemoglobin Alc is denatured, the newly exposed epitope becomes an effective competitor for the linear glycopeptide-enzyme conjugate.

EXAMPLE 4: Comparison of Antibody Specificity - Sheep Polyclonal vs. Mouse Monoclonal Response

A sheep is immunized, 5 sites, IM in Freund's complete adjuvant with the glycopeptide-KLH conjugate (4 mg) of example 1(b). Boost injections are done similarly after 30 days and 60 days. The 60 day boost is in Freund's incomplete adjuvant. Preimmune serum, and immune serum is titered for its $A_{1c}$ and Ao specificity in an ELISA assay as described in Example 1(c). The results, (see Fig. 8) show that the synthetic glycopeptide stimulates an immune response against human hemoglobin, but that the immunoglobulins are not specific for $A_{1c}$ hemoglobin. In contrast, mouse monoclonal antibodies for $A1_c$ are quite specific for $A_{1c}$ when measured in the same assay (the ELISA assay of Example 1c - see Fig. 9). Attempts to immunoaffinity purify antibody specific for $A1_c$ from the sheep antiserum were not successful.

EXAMPLE 5: Preparation of Hapten-Label Conjugates

A conjugate of the GLYCOPEPTIDE 1 (HRP) was prepared. The hapten-HRP conjugate was prepared by reacting 15 mg horseradish peroxidase (HRP) with a 10 x molar excess of MBS (see Example 1b) in 50 mM sodium phosphate, 1mM EDTA, pH 7.0. The MBS-HRP conjugate was purified by gel filtration (using the above buffer) and 0.34 mg of the glycopeptide hapten (PEPTIDE 1) was added. The final hapten-HRP conjugate was purified by gel filtration on HPLC and was used at a dilution of 1:1000 - 1:100,000 in the competitive immunoassay of Example 3.

EXAMPLE 6: Strip Immunoassay for Hb Alc

a) 1 mg of the monoclonal antibody of Example 1(c) in 0.1 molar sodium borate buffer, pH 8.5 can be mixed with a 200-fold molar excess of fluorescein isothiocyanate (FITC) and reacted for 30 minutes at room temperature. The fluorescein labeled monoclonal antibody can be purified by gel filtration.

b) A strip (polystyrene, cellulose, etc.) carrying COOH groups is dipped into 0.5 ml of unknown denatured hemolysate, pH 7.5. The strip is rinsed with buffer at pH 7.5 and immersed into the fluorescent monoclonal antibody of (a) in buffered solution, for 5 minutes at room termperature. The strip is again rinsed and the degree of fluorescence of the strip indicates the degree of Alc Hb in the unknown sample.

EXAMPLE 7: Coupling Monoclonal Antibody to Reagent Strip and Its Use in an Immunoassay

Whatman #1 filter paper (7 cm) is placed in 20 ml ice-cold d-$H_2O$ and the pH of the solution is adjusted to between 10.5 - 11.5 with 5M NaOH. Solution is monitored continuously throughout the activation and the pH is maintained between 10.5 - 11.5 with dropwise addition of 5M NaOH. A small stir bar is placed in the bottom of the beaker containing the filter paper. The beaker is then placed in an ice filled petri dish which is

placed on a magnetic stirrer. 1 gram of solid BrCN added to the beaker and this is incubated with stirring for 20 minutes (on ice). Filter paper is removed from the solution and washed in 100 ml ice-cold distilled water (d-$H_2O$). It is then washed in ice-cold 0.2M $Na_2H\ PO_4$-citric acid buffer, pH 6.8. Antibody (1 mg/ml in 0.2M $Na_2HPO_4$-citric acid buffer, pH 6.8) is added and the coupling of antibody is allowed to proceed for 1 hour. Ethanolamine (10 ml of a 1 mM solution) is added to block unreacted sites (15 minutes) and the paper washed with phosphate buffered saline (PBS, 10 mM $NaH_2PO_4$, 140 mM NaCl, pH 7.5) to remove unreacted components.

This reagent strip is dipped into a standardized quantity of unknown denatured hemolysate containing a labeled competitor of the antibody binding for Alc hemoglobin. A convenient competitor is the glycopeptide (GLYCOPEPTIDE 1) covalently coupled to horseradish peroxidase (HAPTEN-HRP). The strip is removed and rinsed with PBS. The amount of hemoglobin bound to the strip is measured (the quantity is inversely proportional to the Alc in the sample and the Alc hemoglobin can be quantitated by comparison to standard samples.

EXAMPLE 8: Enzyme linked immunosorbent assay for Alc

A fixed volume o denatured blood hemolysate (100 $\mu$l) is added to polystyrene microtiter plates and allowed to bind at room temperature for 60 minutes. The place is washed four times in PBS containing 0.05% Tween-20 (PBST). The monoclonal antibody (coupled to horseradish peroxidase) is added (100 $\mu$l, 1 $\mu$g/ml) in PBST and reacted for 30 minutes at room temperature. The excess antibody is removed with 4 washes of PBST. The substrate (o-phenylenediamine, 2.2 mM) and $H_2O_2$ (0.012%) in PBS are added and the reaction product measured at 492 nm. The color intensity reflects the quantity of Alc present in the hemolysate when compared to standard values.

EXAMPLE 9: Radioimmunoassay for Alc

One hundred microliters of denatured blood hemolysate (220 n moles hemoglobin) is mixed with 7 n moles iodinated
Glyco-Val-His-Leu-Thr-Pro-Glu-Glu-Lys-Tyr-Tyr-Cys (500,000 cpm/7 n moles). A monoclonal antibody is added in a quantity sufficient to bind to 50% of the glucosylated peptide if the blood hemolysate contains the normal (approximately 3%) Alc hemoglobin. Higher hemoglobin Alc values compete for the peptide thereby reducing the total number of counts bound by the antibody. The antibody can be recovered by immunoprecipitating with a second antibody (rabbit anti mouse IgG) or by adsorption onto protein A coated particles. The iodinated peptide bound to the antibody can be quantitated in a gamma isotope counter and reflects the quantity of Alc present in the blood hemolysate when compared to standards.

## Claims

**1.** A monoclonal antibody, or a fragment thereof comprising an antibody combining site, characterized in that it binds specifically to the glucosylated N-terminal peptide sequence in the beta-subunit of human hemoglobin.

**2.** The monoclonal antibody or fragment thereof of claim 1 characterized in that it binds specifically to a glucosylated peptide residue of the formula:
Glyco-(NH)Val-His-AA-
wherein Glyco-(NH)Val represents a nonenzymatically glucosylated valine residue and AA is a bond or one or more additional amino acid residues.

**3.** The monoclonal antibody or fragment thereof of Claim 2 characterized in that AA is a sequence of from 1 to 12 amino acids corresponding to the N-terminus of the beta-subunit of human hemoglobin.

**4.** The monoclonal antibody or fragment of any of claims 1 to 3 characterized in that it has been raised in a mouse against an immunogen comprising a glucosylated peptide chemically linked to an immunogenic carrier material, the glucosylated peptide having at least 2 amino acid units corresponding to the N-terminus of the beta-subunit of hemoglobin, or a denatured form of hemoglobin or a fragment thereof comprising the glucosylated N-terminus of the beta-subunit.

5. The monoclonal antibody or fragment of any of claims 1 to 4 which binds specifically to said glucosylated N-terminal peptide sequence upm being exposed sufficiently to provide steric access thereto.

6. The monoclonal antibody or fragment of claim 5 wherein said glucosylated peptide sequence is exposed to the antibody by physical or chemical denaturation or digestion.

7. The monoclonal antibody or fragment of any of claims 5 and 6 wherein said glucosylated peptide sequence is exposed to the antibody by chemical denaturation by contact with a chaotropic agent, preferably guanidine, urea, potassium - thiocyanate, or detergent.

8. The monoclonal antibody or fragment of any of claims 1 to 4 which binds specifically to said glucosylated N-terminal peptide sequence or hemoglobin that has been adsorbed to a solid surface, preferably made of polystyrene or cellulose.

9. A hybridoma cell line which secrets monoclonal antibody which binds specifically to the glucosylated N-terminal peptide sequence in the beta-subunit hemoglobin.

10. Monoclonal antibodies against hemoglobin Alc produced by hybridomas having the ATCC identification numbers HB 8639 or HB 8869.

**Patentansprüche**

1. Monoklonaler Antikörper oder Fragment davon, das eine Antikörper-Bindungsstelle umfasst, dadurch **gekennzeichnet,** dass er spezifisch an die glucosylierte N-terminale Peptidsequenz in der $\beta$-Untereinheit von menschlichem Hämoglobin bindet.

2. Monoklonaler Antikörper oder Fragment davon nach Anspruch 1, dadurch **gekennzeichnet,** dass er spezifisch an einen glucosylierten Peptidrest der Formel
Glyco-(NH)Val-His-AA-
bindet, worin Glyco(NH)Val einen nicht-enzymatisch glucosylierten Valinrest darstellt und AA eine Bindung oder ein oder mehrere weitere Aminosäurereste ist.

3. Monoklonaler Antikörper oder Fragment davon nach Anspruch 2, dadurch **gekennzeichnet,** dass AA eine Sequenz von 1 bis 12 Aminosäuren ist, die dem N-Terminus der $\beta$-Untereinheit von menschlichem Hämoglobin entspricht.

4. Monoklonaler Antikörper oder Fragment nach einem der Ansprüche 1 bis 3, dadurch **gekennzeichnet,** dass er in einer Maus gegen ein Immunogen erzeugt wurde, das ein glucosyliertes Peptid, das chemisch an ein immunogenes Trägermaterial gebunden wurde, umfasst, wobei das glucosylierte Peptid mindestens zwei Aminosäureeinheiten hat, die dem N-Terminus der $\beta$-Untereinheit von Hämoglobin entsprechen, oder einer denaturierten Form von Hämoglobin oder einem Fragment davon, das den glucosylierten N-Terminus der $\beta$-Untereinheit umfasst, entsprechen.

5. Monoklonaler Antikörper oder Fragment nach einem der Ansprüche 1 bis 4, das spezifisch an die glucosylierte N-terminale Peptidsequenz bindet, wenn sie hinreichend exponiert wird, so dass ein sterischer Zugang ermöglicht wird.

6. Monoklonaler Antikörper oder Fragment nach Anspruch 5, dadurch **gekennzeichnet,** dass die glucosylierte Peptidsequenz an den Antikörper durch physikalische oder chemische Denaturierung oder Verdauung exponiert wird.

7. Monoklonaler Antikörper oder Fragment nach einem der Ansprüche 5 und 6, dadurch **gekennzeichnet,** dass die glucosylierte Peptidsequenz an den Antikörper durch chemische Denaturierung durch Kontakt mit einem chaotropen Agens, vorzugsweise Guanidin, Harnstoff, Kaliumthiocyanat oder ein Detergenss exponiert wird.

8. Monoklonaler Antikörper oder Fragment nach einem der Ansprüche 1 bis 4, der spezifisch an die besagte glucosylierte N-terminale Peptidsequenz oder Hämoglobin bindet, das an eine feste Oberfläche adsorbiert wurde, welche vorzugsweise aus Polystyrol oder Cellulose besteht.

9. Hybridoma-Zellinie, welche monoklonale Antikörper ausscheidet, die spezifisch an die glucosylierte N-terminale Peptidsequenz in der $\beta$-Untereinheit von Hämoglobin bindet.

10. Monoklonale Antikörper gegen Hämoglobin-$A_{1c}$, hergestellt von Hybridomas mit den ATCC-Registrationsnummern HB 8639 oder HB 8869.

**Revendications**

1. Anticorps monoclonal, ou un de ses fragments comprenant un site de fixation de l'anticorps, caractérisé en ce qu'il se lie spécifiquement à la séquence peptidique N-terminale glucosylée dans la sous-unité bêta de l'hémoglobine humaine.

2. Anticorps monoclonal ou un de ses fragments suivant la revendication 1, caractérisé en ce qu'il se lie spécifiquement à un résidu peptidique glucosylé, de formule :
Glyco-(NH)Val-His-AA-
dans laquelle le groupe Glyco-(NH)Val représente un résidu valine glucosylé par voie non enzymatique et AA représente une liaison ou bien un ou plusieurs résidus d'amino-acides supplémentaires.

3. Anticorps monoclonal ou un de ses fragments suivant la revendication, caractérisé en ce que AA représente une séquence de 1 à 12 amino-acides correspondant à l'extrémité N-terminale de la sous-unité bêta de l'hémoglobine humaine.

4. Anticorps monoclonal ou fragment suivant l'une quelconque des revendications 1 à 3, caractérisé en ce qu'il a été engendré chez une souris contre un immunogène comprenant un peptide glucosylé lié chimiquement à une matière immunogène de support, le peptide glucosylé ayant au moins deux motifs amino-acide correspondant à l'extrémité N-terminale de la sous-unité bêta de l'hémoglobine, ou une forme dénaturée d'hémoglobine ou un de ses fragments comprenant l'extrémité N-terminale glucosylée de la sous-unité bêta.

5. Anticorps monoclonal ou fragment suivant l'une quelconque des revendications 1 à 4, qui se lie spécifiquement à la séquence peptidique N-terminale glucosylée par une exposition suffisante pour permettre un accès stérique à cette séquence.

6. Anticorps monoclonal ou fragment suivant la revendication 5, dans lequel la séquence peptidique glucosylée est exposée à l'anticorps par une dénaturation physique ou chimique ou une digestion.

7. Anticorps monoclonal ou fragment suivant l'une quelconque des revendications 5 et 6, dans lequel la séquence peptidique glucosylée est exposée à l'anticorps par dénaturation chimique par contact avec un agent chaotropique, de préférence la guanidine, l'urée, le thiocyanate de potassium ou un détergent.

8. Anticorps monoclonal ou fragment suivant l'une quelconque des revendications 1 à 4, qui se lie spécifiquement à la séquence peptidique N-terminale glucosylée de l'hémoglobine qui a été adsorbée sur une surface solide, constituée de préférence d'un polystyrène ou de cellulose.

9. Lignée cellulaire d'hybridomes qui sécrète un anticorps monoclonal qui se lie spécifiquement à la séquence peptidique N-terminale glucosylée dans la sous-unité bêta de l'hémoglobine.

10. Anticorps monoclonaux contre l'hémoglobine Alc, produits par des hybridomes ayant le numéro d'identification ATCC HB 8639 ou HB 8869.

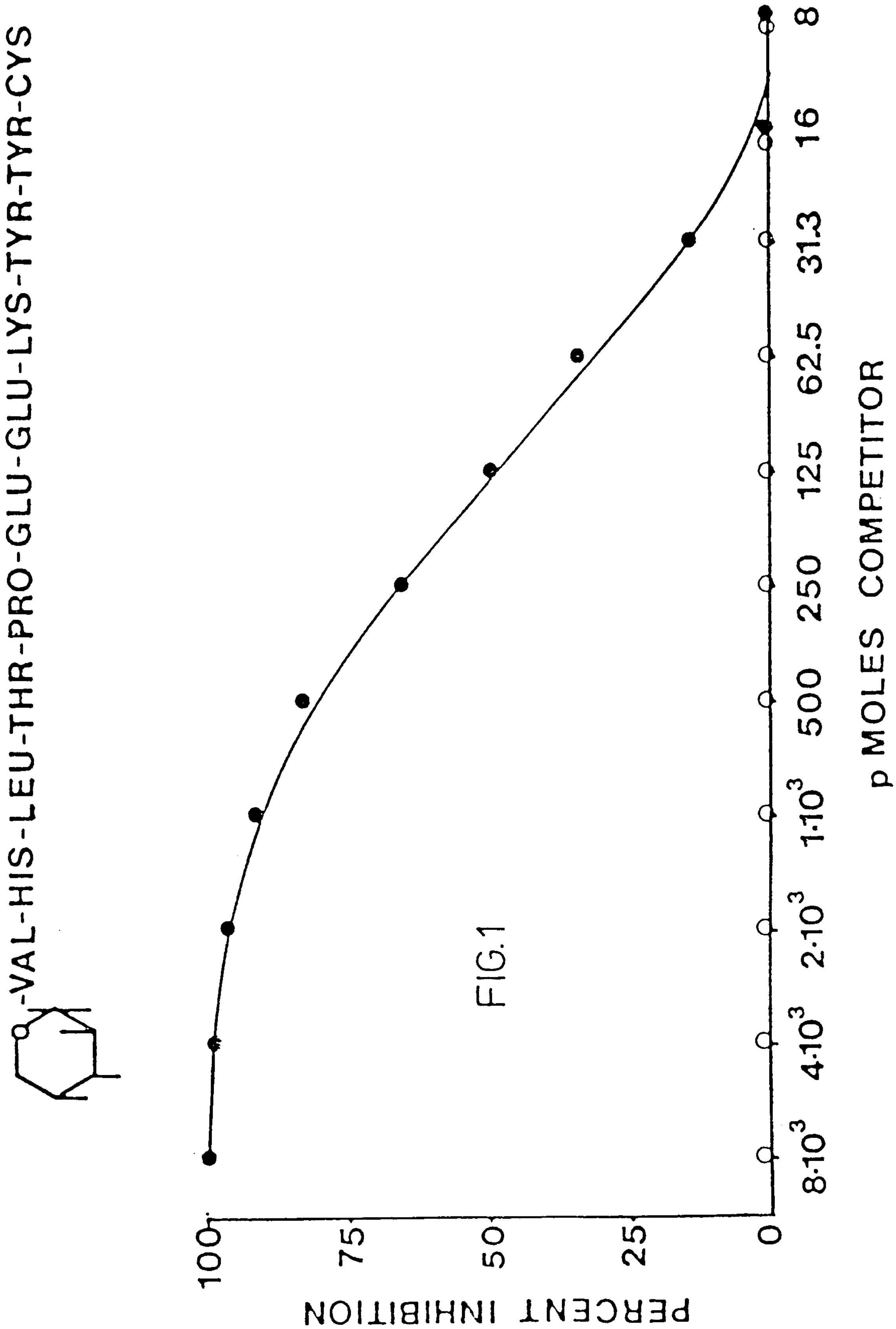
-VAL-HIS-LEU-THR-PRO-GLU-GLU-LYS-TYR-TYR-CYS
PERCENT INHIBITION
100
75
50
25
0
8·10³
4·10³
2·10³
1·10³
500
250
125
62.5
31.3
16
8
p MOLES COMPETITOR

FIG.1

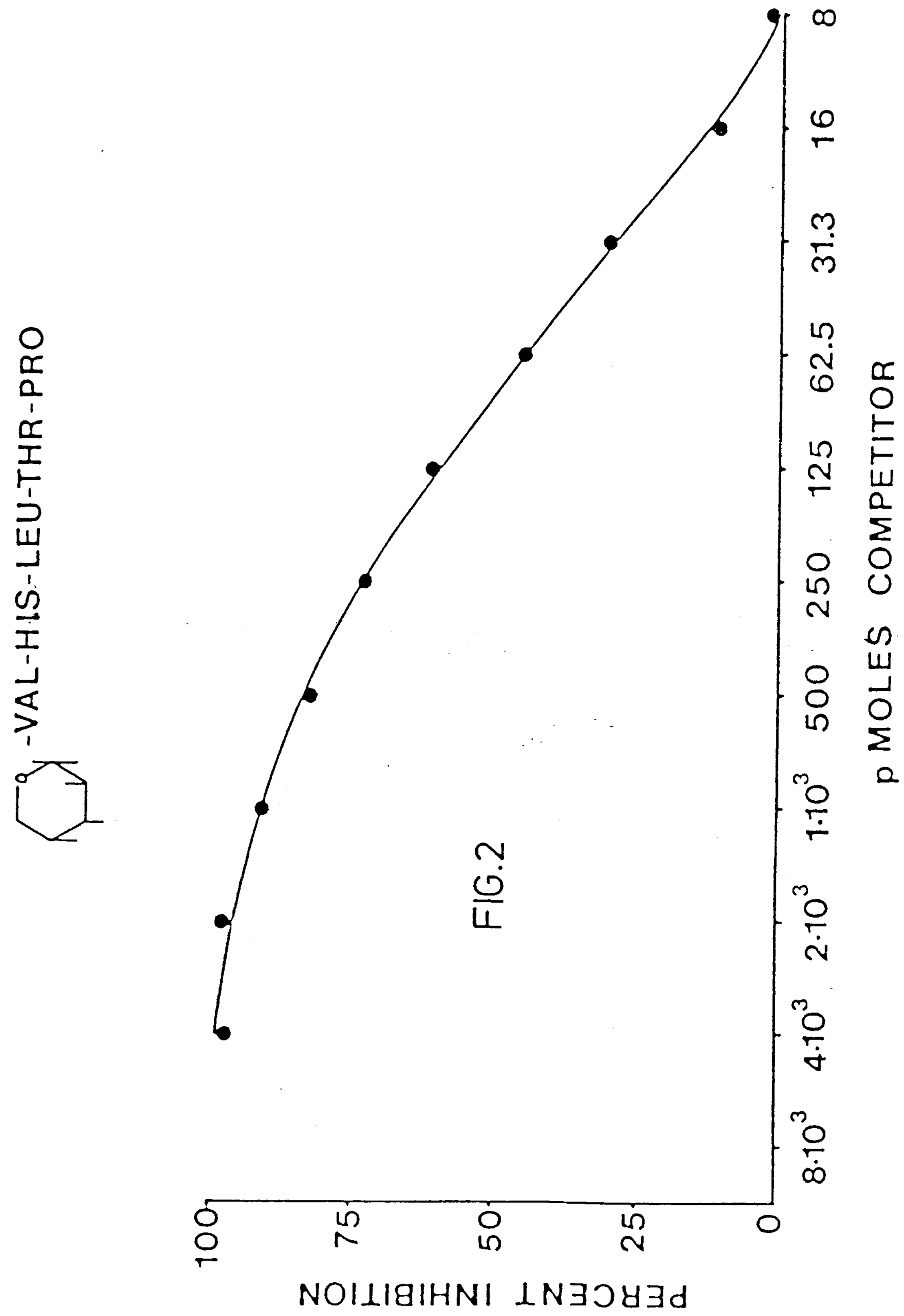
-VAL-HIS-LEU-THR-PRO
PERCENT INHIBITION
100
75
50
25
0
8·10³
4·10³
2·10³
1·10³
500
250
125
62.5
31.3
16
8
p MOLES COMPETITOR

FIG.2

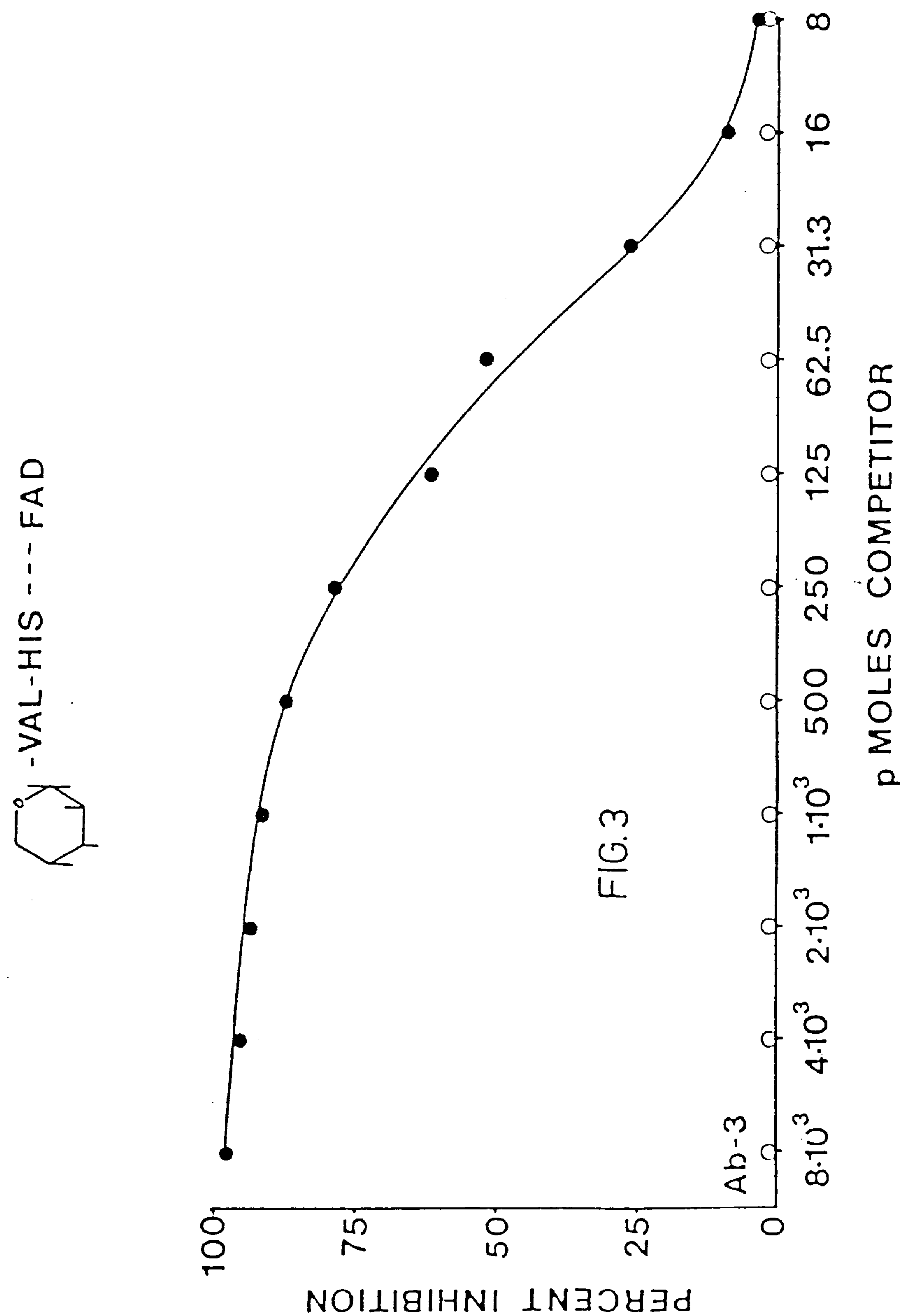
-VAL-HIS --- FAD
Ab-3
PERCENT INHIBITION
100
75
50
25
0
8·10³
4·10³
2·10³
1·10³
500
250
125
62.5
31.3
16
8
p MOLES COMPETITOR

FIG.3

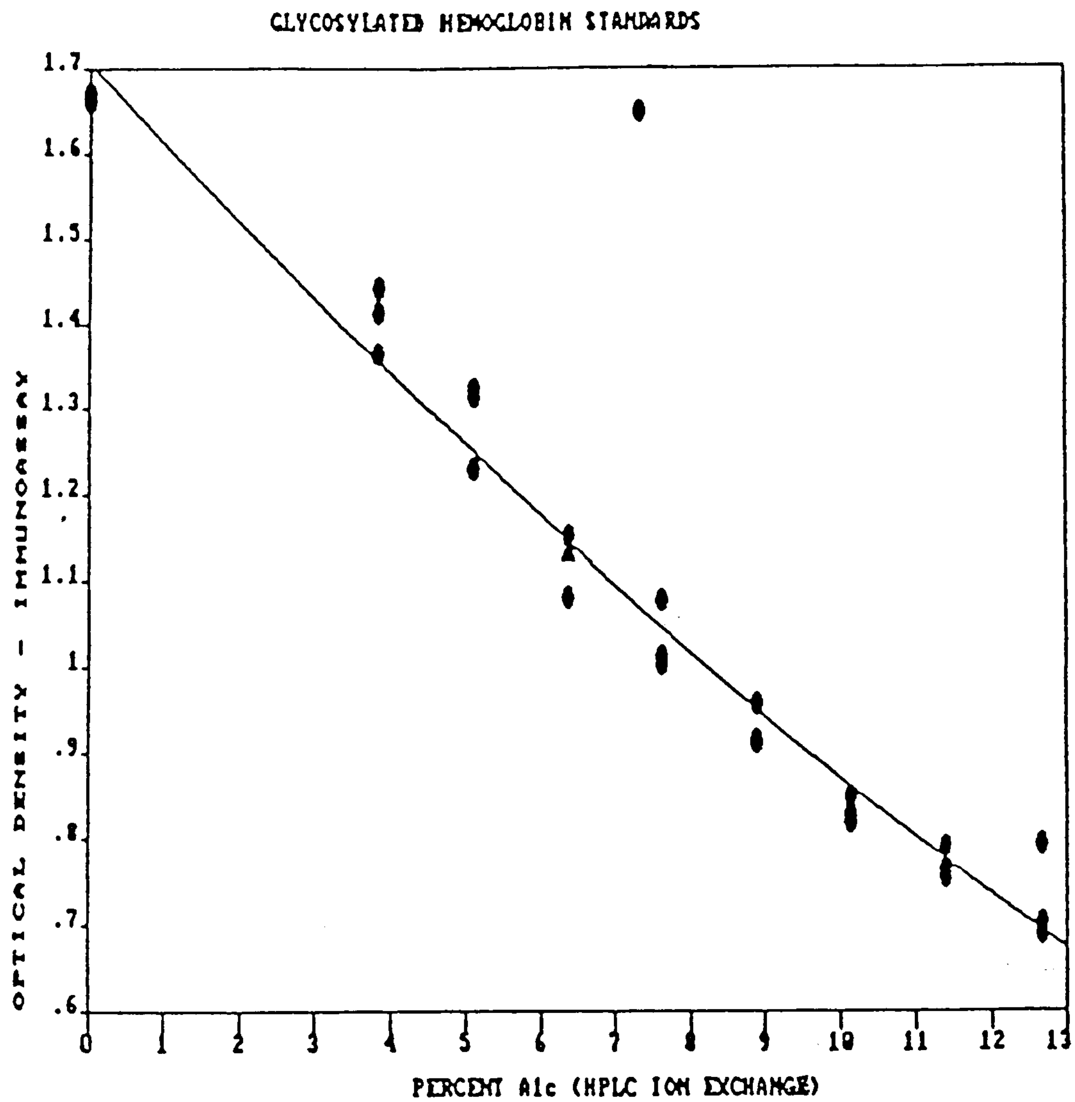
GLYCOSYLATED HEMOGLOBIN STANDARDS
1.7
1.6
1.5
1.4
1.3
1.2
1.1
1
.9
.8
.7
.6
0
1
2
3
4
5
6
7
8
9
10
11
12
13
PERCENT A1c (HPLC ION EXCHANGE)
OPTICAL DENSITY - IMMUNOASSAY

FIG. 4

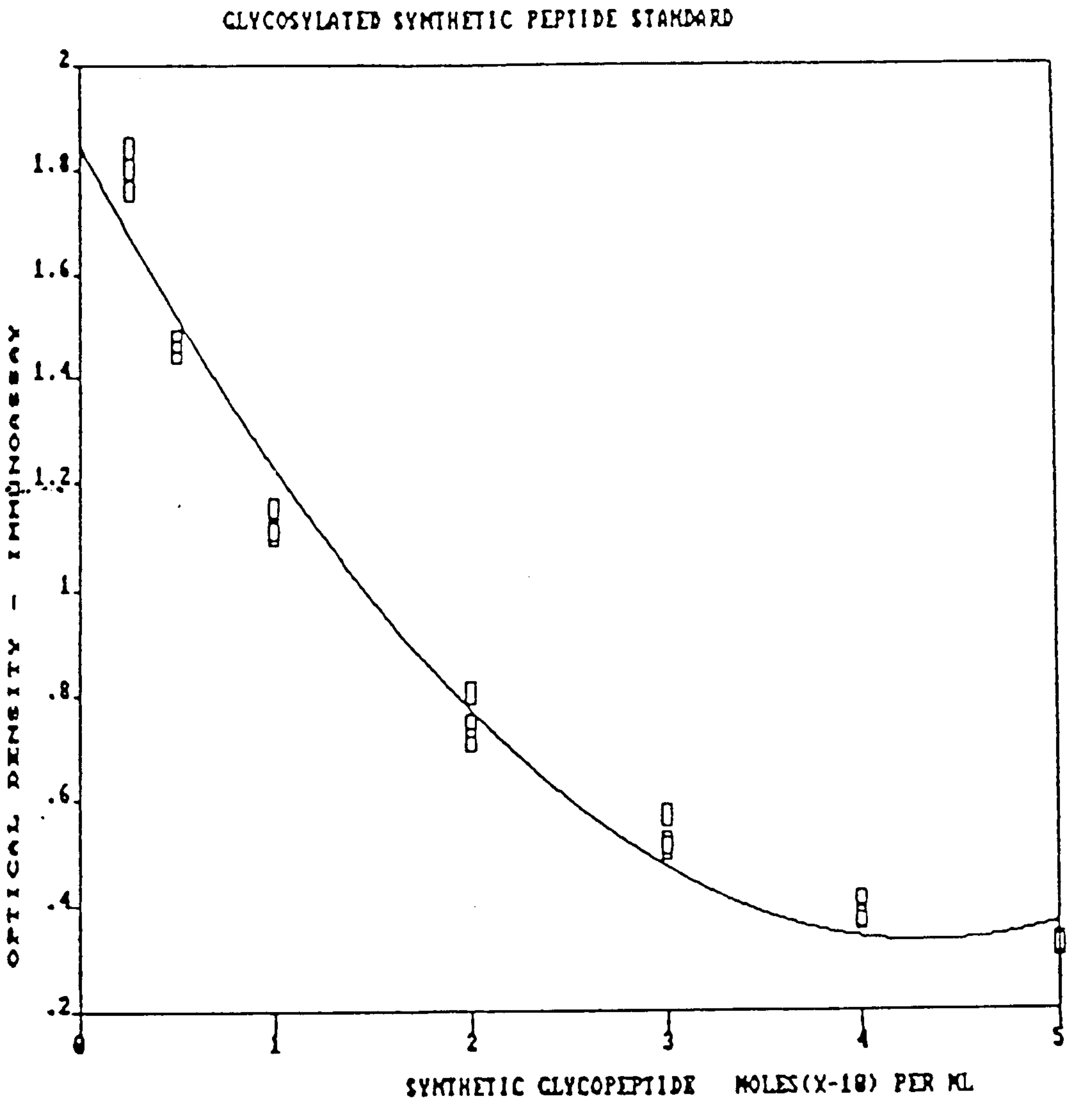
GLYCOSYLATED SYNTHETIC PEPTIDE STANDARD
OPTICAL DENSITY - IMMUNOASSAY
2
1.8
1.6
1.4
1.2
1
.8
.6
.4
.2
0
1
2
3
4
5
SYNTHETIC GLYCOPEPTIDE MOLES(X-18) PER ML

FIG.5

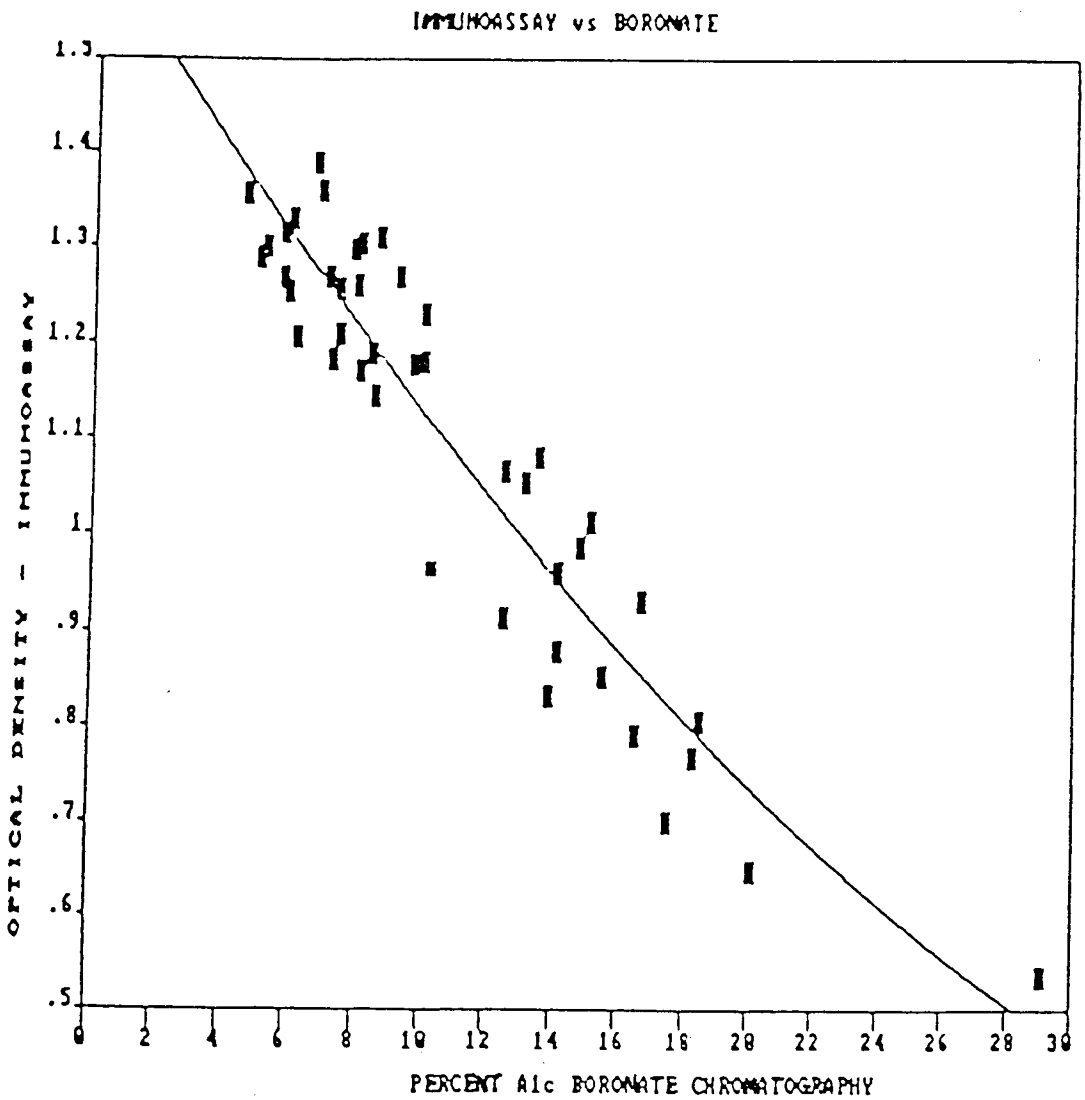
IMMUNOASSAY vs BORONATE
OPTICAL DENSITY - IMMUNOASSAY
1.5
1.4
1.3
1.2
1.1
1
.9
.8
.7
.6
.5
0
2
4
6
8
10
12
14
16
18
20
22
24
26
28
30
PERCENT A1c BORONATE CHROMATOGRAPHY

FIG. 6

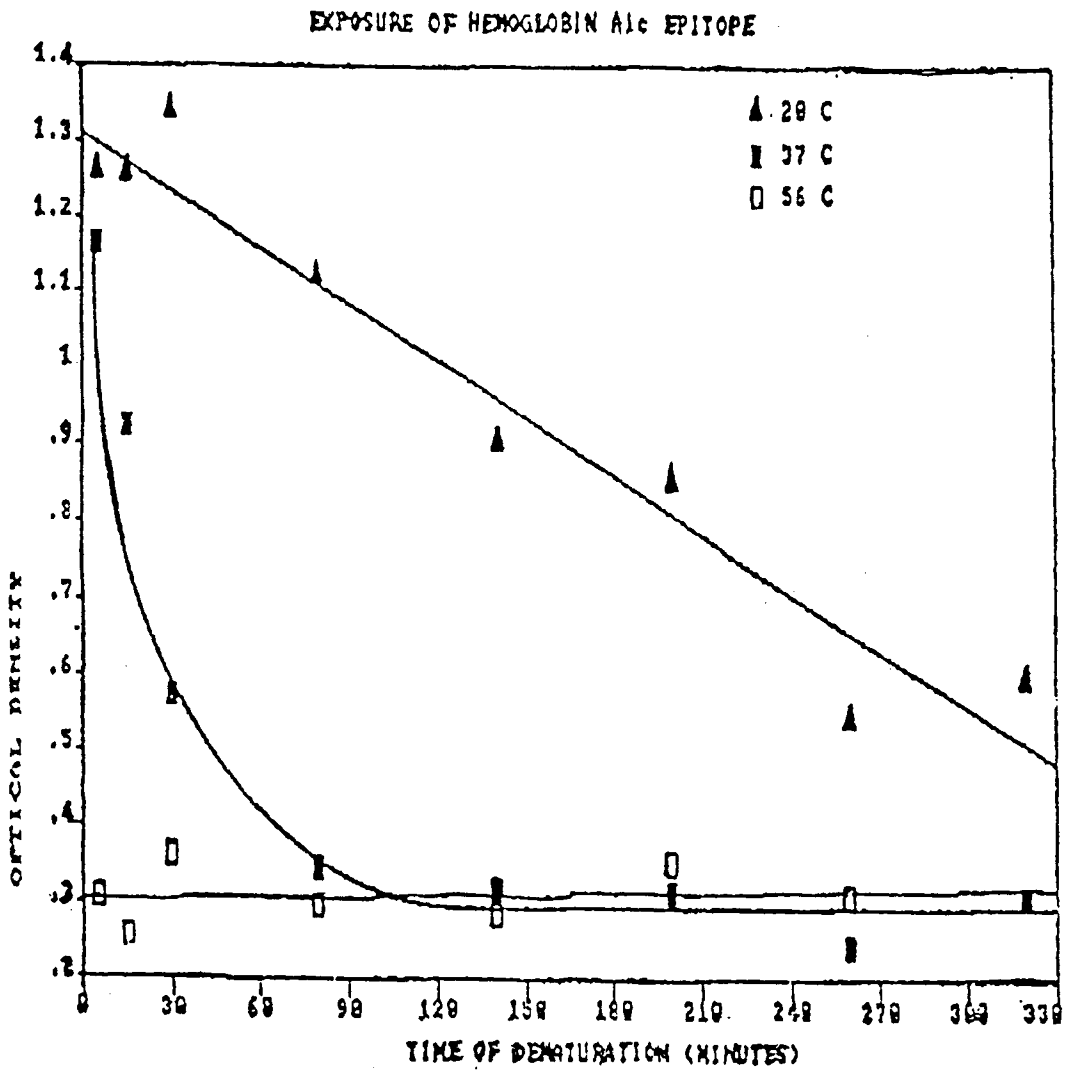
EXPOSURE OF HEMOGLOBIN A1c EPITOPE
OPTICAL DENSITY
TIME OF DENATURATION (MINUTES)
28 C
37 C
56 C

FIG. 7

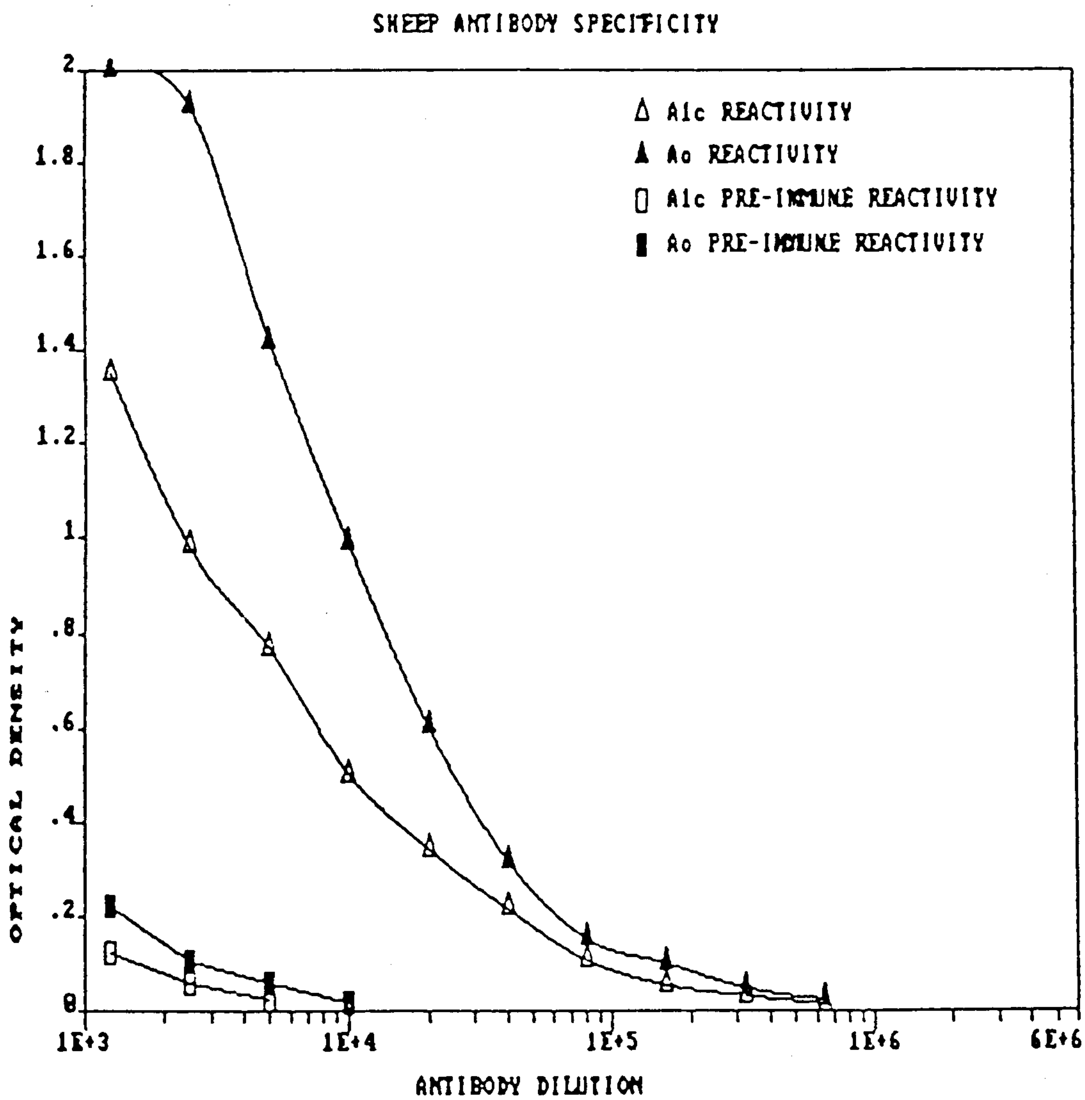
SHEEP ANTIBODY SPECIFICITY
Δ A1c REACTIVITY
▲ Ao REACTIVITY
□ A1c PRE-IMMUNE REACTIVITY
■ Ao PRE-IMMUNE REACTIVITY
OPTICAL DENSITY
2
1.8
1.6
1.4
1.2
1
.8
.6
.4
.2
0
1E+3
1E+4
1E+5
1E+6
6E+6
ANTIBODY DILUTION

FIG. 8

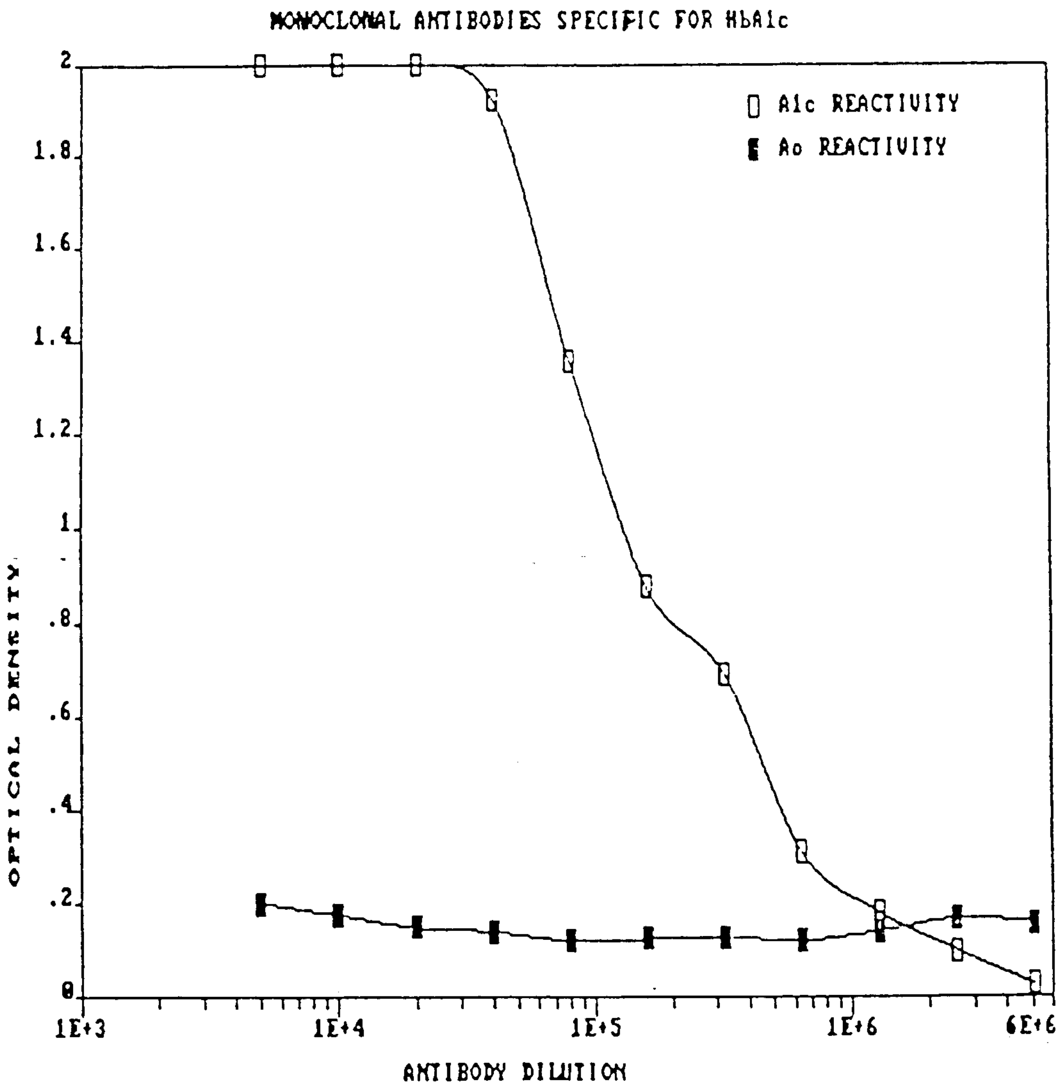
MONOCLONAL ANTIBODIES SPECIFIC FOR HbA1c
A1c REACTIVITY
Ao REACTIVITY
OPTICAL DENSITY
2
1.8
1.6
1.4
1.2
1
.8
.6
.4
.2
0
1E+3
1E+4
1E+5
1E+6
6E+6
ANTIBODY DILUTION

FIG. 9